(19) Europäisches Patentamt
European Patent Office
Office européen des brevets

(11) Numéro de publication: **0 169 767 B1**

# (12) FASCICULE DE BREVET EUROPEEN

(45) Date de publication du fascicule du brevet: 18.04.90

(51) Int. Cl.$^5$: C 12 N 11/02, C 12 Q 1/66

(21) Numéro de dépôt: 85401306.7

(22) Date de dépôt: 27.06.85

## (54) Membrane porteuse de la luciférase pour le dosage de l'ATP et son procédé de production.

(30) Priorité: 27.06.84 FR 8410119

(43) Date de publication de la demande:
29.01.86 Bulletin 86/05

(45) Mention de la délivrance du brevet:
18.04.90 Bulletin 90/16

(84) Etats contractants désignés:
BE CH DE GB IT LI NL SE

(56) Documents cités:
EP-A-0 019 786 US-A-4 411 999

TRAC, TRENDS IN ANALYTICAL CHEMISTRY, vol. 2, no. 11, novembre 1983, pages 244-247, Cambridge, GB; L.J. KRICKA et al.: "Immobilized bioluminescent enzymes"

CHEMICAL ABSTRACTS, vol. 97, no. 11, 13 septembre 1982, page 366, no. 87825x, Columbus, Ohio, US; N.N. UGAROVA et al.: "Immobilization of luciferase from the firefly Luciola mingrelica - catalytic properties and stability of the immobilized enzyme", & ENZYME MICROB. TECHNOL. 1982, 4(4), 224-8

BIOTECHNOLOGY & BIOENGINEERING, vol. 27, no. 3, mars 1985, pages 232-237, New York, US; L.J. BLUM et al.: "Collagen strip with immobilized luciferase for ATP bioluminescent determination"

(73) Titulaire: Etablissement Public dit: CENTRE NATIONAL DE LA RECHERCHE SCIENTIFIQUE (CNRS)
15, Quai Anatole France
F-75007 Paris (FR)

(72) Inventeur: Vijayalakshmi, Mookambeswaren Arunachalam
Les Tulipes 11 square du Commandant Fournaise
F-60200 Compiegne (FR)
Inventeur: Rajgopal, Sunanda
4 rue Pierre et Marie Curie Appartement 38
F-60200 Compiegne (FR)

(74) Mandataire: Corre, André et al
Cabinet Corre A. 17, rue Pasteur
F-92300 Levallois (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 169 767 B1

## Description

La présente invention est relative à une membrane porteuse de la luciférase pour, notamment, le dosage de l'acide adénosine-triphosphorique, ou ATP, ainsi qu'à un procédé pour sa production.

La luciférase est une enzyme généralement extraite de la luciole (Photinus pyralis). Récemment elle est l'objet d'un regain d'intérêt du fait de sa spécificité pour l'A.T.P. elle permet en ffet de doser cet acide ou ses sels dans de nombreux produits alimentaires. Par exemple, en dosant l'A.T.P. présent dans le lait, on peut effectuer le dosage microbien ou celui de la créatinine en analyse diasique. Le dosage de l'A.T.P. est réalisé par photométrie puisque sa réaction avec l'enzyme produit une émission de potons selon les deux équations réactionnelles suivantes:

$$E+LH_2+ATP \xrightarrow{Mg^{++}} ELH_2-AMP+PP$$

$$E \cdot LH_2-AMP+O_2 \rightarrow E+P+CO_2+AMP+\text{photons}$$

dans lesquelles E représente la luciférase, $E \cdot LH_2-AMP$ le complexe luciférine adenylate, P le produit de la réaction d'oxydation produisant l'oxyluciférine et PP le pyrophosphate.

L'intensité de la bioluminescence mesurée est directement proportionnelle à la quantité d'A.T.P. présente dans le produit considéré.

Mais la mise en oeuvre de cette méthode a été longtemps freinée par le manque de stabilité de la luciférase. Aussi un certain nombre de méthodes pour cet enzyme a t-il été étudié. Ainsi, on réalis a son piégeage dans un gel d'albumine additionné de glutaraldéhyde, ou dans un gel polyacrylamide, son adsorption sur ce dernier gel ou son immobilisation sur différents supports de polysaccharides tels que: AH-Sepharose 4B ou CH-Sepharose 4B en utilisant un carbodiimide, ou CH-Sepharose 4B en utilisant le réactif de Woodward, ou encore son immobilisation sur des supports polysaccharidiques activés par un bromure de cyanure (CNBr). Seule cette dernière méthode a été considérée comme satisfaisante car elle permet de conserver 20% de l'activité de la luciférase, taux nettement supérieur à tous ceux obtenus par les autres méthodes. De ce fait, la luciférase ainsi immobilisée est produite à un coût relativement élevé pouvant être considéré comme prohibitif pour des applications industrielles. Par ailleurs, elle ne présente pas une stabilité réellement satisfaisante.

Aussi un des buts de la présente invention est-il de fournir une luciférase immobilisée à un coût attractif afin d'en permettre une grande utilisation industrielle.

Un autre but de l'invention est de fournir une telle luciférase qui est stable à la température et dans le temps.

Un but supplémentaire de l'invention est de fournir un procédé pour la préparation d'une telle luciférase immobilisée qui est simple et peut être réalisé à une échelle industrielle.

Ces buts, ainsi que d'autres qui apparaîtront par la suite, sont atteints par la luciférase immobilisée sur une membrase constituée par de la gélatine de peau de porc.

La présente invention est également relative à un procédé pour fabriquer une telle membrane porteuse de luciférase selon lequel on dissout la gelatine de peau de porc dans un tampon faiblement acide afin d'obtenir une membrane homogène, on effectue la réticulation de cette membrane homogène, à l'aide d'une solution de glutaraldéhyde à un pH faiblement acide, et on sature la membrane ainsi obtenue par une quantité déterminée de luciférase.

De préférence, le tampon faiblement acide est constitué par une solution de phosphate de sodium dont le pH est de 6,8.

Avantageusement la solution obtenue par dissolution de la gelatine de peau de porc dans le tampon comprend 6% en poids de cette gelatine.

De préférence, on réalise une membrane homogène à partir de cette solution, en l'étalant, après une incubation courte à une température comprise entre 25°C et 50°C, sur un support convenable dont elle sera retirée après séchage.

Avantageusement, l'incubation est effectuée pendant une heure à une température de 37°C.

De préférence, le support convenable est constitué par un film de polycarbonate.

Avantageusement, la réticulation de la membrane homogène est effectuée par une solution à 0,5% en volume de glutaraldéhyde.

De préférence, le pH de la solution de glutaraldéhyde est le même qui celui du tampon phosphorique.

Avantageusement, la saturation par la luciférase de la membrane est effectuée en présence de dithiothréitol (D.T.T.).

La description qui va suivre et qui ne présente aucun caractère limitatif permettra à l'homme du métier de mieux comprendre la présente invention ainsi que de mettre en évidence les avantages de celle-ci.

La luciférase est préparée, selon une méthode connue, à partir d'abdomens desséchés de lucioles (Photinus pyralis par exemple) dans un tampon à 0,1 m d'arsénate dont le pH est de 7,4, comme cela est décrit par S. Rajgopal et al. dans J. of Chromatography 1982, n° 243, pages 164—167. L'extrait ainsi obtenu est dialysé pendant 24 heures dans du tris-acétate à 0,02 M (pH=7,8) en présence de 1 mM d'acide éthylène-diamino-tétracétique (EDTA) et avec trois changements du tampon.

La Demanderesse a choisi d'utiliser la gélatine de peau de porc pour réaliser une membrane sur laquelle sera immobilisée la luciférase.

Dans un premier temps, on dissout la gélatine de peau de porc dans un tampon de phosphate de sodium à 0,02 M (pH=6,8) de façon à obtenir une solution dans laqelle la concentration en gélatine est de 6% en poids. Cette solution est ensuite incubée pendant une heure à 37°C. Dans un second temps on étend la solution obtenur sur un film de polycarbonate, matériau convenant du fait

de sa non-adhérence: par exemple sur une surface de 15 $cm^2$ d'un tel film, on disperse 15 ml de cette solution. Puis on laisse sécher pendant environ une dizaine d'heures. Une membrane homogène se forme ainsi sur ce film de polycarbonate dont elle se détache aisément.

La réticulation de cette membrane homogène est réalisée en trempant des bandes ou des carrés de celle-ci pendant un court laps de temps, de l'ordre de cinq minutes, dans un tampon identique au précédent qui comprend en outre 0,5% en volume de glutaraldéhyde. La membrane ainsi réticulée est lavée plusieurs fois avec ce même tampon afin d'éliminer l'excès de glutaraldéhyde.

L'immobilisation de la luciférase sur la membrane rèticulée est effectué par saturation par une quantité déterminée de luciférase de préférence en présence de dithiothréitol (D.T.T.). Après une heure à température ambiante, on lave cette membrane réticulée sur laquelle est immobilisée de la luciférase, avec le tempon déjà décrit cidessus.

Les essais suivants ont permis de déterminer les différents paramètres du procédé pour obtenir de la luciférase immobilisé sur une membrane protéinique.

1. Effet du glutaraldéhyde

Différentes membranes homogènes ont été réticulées avec des tampons de phosphate de sodium à 0,02 M (pH 6,8) comprenant des concentrations de glutaraldéhyde variant entre 0,5 et 5,0%. Les membranes réticulées ainsi obtenues -en fait des disques de celles-ci- ont été plongées dans une solution de luciférase en présence de DTT pendant une heure à température de +4°C. Sur chaque centimètre carré ont été immobilisés (500 µl d'enzyme en présence de 100 µl de DTT (9,5 mg de DTT dans 10 ml de morpholinopropane-sulfonate ou MOPS).

Il a été ainsi montré que l'activité maximum de la luciférase est obtenue avec une concentration du glutaraldehyde de 0,5%. Cette activité diminue alors que la concentration en glutaraldéhyde augmente jusqu'à 1% puis devient supérieure à 2%. Entre une concentration comprise entre 1% et 2%, l'activité enzymatique de la luciférase croit linéairement.

Il y a lieu de rappeler que la luciférase en solution est totalement inactivée par le glutaraldéhyde en une heure de temps et que le dithiothréitol n'a aucune influence sur cette vitesse d'inactivation.

2) Effet du temps de contact de la luciférase avec des membranes réticulées.

On laisse des disques de membranes réticulées en présence de 0,5% de glutaraldéhyde en contact avec de la luciférase pendant différentes durées. Le temps de contact maximum ainsi mis en évidence est de une heure. Pour des durées différentes, on a pu constater des pertes d'activité significatives.

Dans le cas de la luciférase en solution, on constate qu'au bout d'une heure, cette solution présente une activité enzymatique résiduelle de 10% par rapport à celle de la solution fraîchement préparée. Si cette solution comporte du DTT, cette activité résiduelle est de 50%.

3) Effet du pH lors de la réticulation

Des disques de membrane homogène ont été placés dans une solution à 5% de glutaraldéhyde dans un tampon de 0,02 M de phosphate de sodium pendant une heure à +4°C. On a constaté que l'activité enzymatique de la luciférase immobilisée ultérieurement sur de telles membranes est maximum lorsque le pH de tampon est de 6,8.

Certes il est connu que la réticulation en présence de glutaraldéhyde est favorisée par un pH acide: elle est en particulier maximum à pH 5. Par contre, l'activité maximum de la luciférase en solution est à pH 7,8. L'expérience a montré que le dosage de l'activité de la luciférase immobilisée sur une membrane réticulée à pH 5 est pratiquement impossible.

4) Effect de la température lors de l'immobilisation

La température optimum d'immobilisation correspond à l'activité enzymatique maximum, a été déterminée en faisant varier la température lors de la mise en contact de disques de membranes réticulées. Cette température est de 18°—20°C c'est-à-dire la température ambiante.

Les caractéristiques de la luciférase immobilisée selon le procédé de la présente invention ont été mises en évidences par les expériences suivantes.

1) Rendement de l'immobilisation

Environ 30 à 40% de l'activité de la luciférase mise au contact d'une membrane réticulée comme décrite ci-dessus est retendue. Une activité spécifique de 100 à 200 unités par milligramme de membrane a été mesurée. De plus la même membrane peut être utilisée plusieurs fois.

Les disques de membrane après utilisation peuvent être lavés et conservés dans un tampon de 0,02M de phosphate de sodium (pH 6,8) en présence de dithiothréitol afin de préserver leur activité.

L'enzyme immobilisée sur de tels disques et conservée dans ces conditions est stable pendant au moins 16 jours.

Le fait que la membrane préparée selon la présente invention soit utilisable pendant un tel laps de temps, représente un avantage important sur le plan industriel.

2) Stabilité dans le temps

De l'étude précédente ont été déduites les conditions optimales pour chaque paramètre étudié.

La luciférase mise au contact d'une membrane qui a été réticulée en présence de 0,5% de glutaraldéhyde à pH 6,8 pendant une heure à la température ambiante en présence de DTT, présente une activité enzymatique stable: deux heures après la fin de la mise en contact, l'activité

enzymatique est de 95% et se maintient à cette valeur.

Dans le cas de la luciférase en solution, ce taux d'activité n'est que de 54%.

3) Stabilité à la température

En chauffant des disques de membrane réticulée comportant de la luciférase, on remarque une stabilité d'activité enzymatique jusqu'à environ 20—25°C, puis une décroissance relativement lente. La tenue mécanique des disques n'est nullement affectuée par la température.

Lorsque la luciférase est en solution, son activité enzymatique maximum est à 4°C puis décroît très rapidement pour être nulle avant 40°C.

Obtenir une membrane utilisable à la température ambiante représente un grand avantage dans la mesure où aucune précaution particulière d'emploi n'est nécessaire. On peut inclure un détecteur d'ATP utilisant une telle membrane sans devoir fondamentalement modifier une chaîne de contrôle dans l'industrie. De plus, on mesure l'activité d'organismes vivants.

4) Stabilité au pH.

Tant pour l'enzyme immobilisée que pour celle en solution, le pH optimum est de 7,4.

5) Concentration de la luciférase et de l'ATP.

Vis-à-vis de ces deux produits, on a pu remarquer que le support (gélatine de peau de porc) n'a aucune influence sur l'affinité du substrat (luciférine ou ATP) pour la luciférase. Il n'y a donc aucun problème de diffusion de l'enzyme immobilisée à la surface de la membrane et ces produits.

L'immobilisation de la luciférase sur une membrane de gélatine de peau de porc permet donc d'obtenir une membrane facilement utilisable, d'une durée de vie acceptable et présentant une grande spécificité pour l'acide adénoisine-triphosphorique (ATP). En effet, on a pu observer une réponse linéaire du pic d'entensité lumineuse avec des concentrations d'ATP variant entre $10^2$ et $10^6$ picogrammes d'ATP par millilitre de solution.

**Revendications**

1. Membrane porteuse de luciférase pour notamment le dosage de l'acide adénosine-triphosphorique caractérisée par le fait que ladite membrane est constituée par de la gélatine de peau de porc.

2. Procédé pour fabriquer une membrane selon la revendication 1, caractérisé en ce que l'on dissout de la gélatine de peau de porc dans un tampon faiblement acide afin d'obtenir une membrane homogène, que l'on effectue ensuite la réticulation de ladie membrane homogène à l'aide d'une solution de glutaraldéhyde à un pH faiblement acide, et on sature le membrane obtenue par une quantité de luciférase.

3. Procédé selon la revendication 2, caractérisé en ce que le tampon faiblement acide est une solution de phosphate de sodium dont le pH est 6,8.

4. Procédé selon la revendication 2 ou 3, caractérisé en ce que la solution obtenue par dissolution de la gélatine de peau de porc dans ledit tampon comprend 6% en poids de ladite gélatine.

5. Procédé selon l'une des revendications 2 à 4, caractérisé en ce que, après la dissolution de la gélatine de peau de porc, on étale la solution obtenue, après une courte incubation à une température comprise entre 25 et 50°C, sur un support convenable dont elle sera retirée après sèchage.

6. Procédé selon la revendication 5, caractérisé en ce que ladite incubation est effectuée pendant une heure à 37°C.

7. Procédé selon la revendication 5, caractérisé en ce que le support convenable est constitué par un film de polycarbonate.

8. Procédé selon l'une quelconque des revendications 2 à 7, caractérisé en ce que la réticulation de ladite membrane homogène est effectuée par une solution à 0,5% en volume de glutaraldéhyde.

9. Procédé selon la revendication 8, caractérisé en ce que le pH de ladite solution de glutaraldéhyde est le même que celui dudit tampon.

10. Procédé selon l'une quelconque des revendications 2 à 9, caractérisé en ce que la saturation par la luciférase de ladite membrane réticulée est effectuée en présence de dithiothreitol.

**Patentansprüche**

1. Luciferaseträgermembrane hauptsächlich zur Dosierung der Adenosinphosphorsäure, dadurch gekennzeichnet, daß die besagte Membrane aus Schweinshautgelatine besteht.

2. Verfahren zur Herstellung einer Membrane gemäß Anspruch 1, dadurch gekennzeichnet, daß man Schweinshautgelatine in einer leicht sauren Pufferlösung löst, um eine homogene Membrane zu erhalten, daß man anschließend die Vernetzung der besagten homogenen Membrane mit Hilfe einer Glutaraldehyd-Lösung mit einem schwach sauren pH-Wert durchführt und man die erhaltene Membrane mit einer Menge Luciferase sättigt.

3. Verfahren gemäß Anspruch 2, dadurch gekennzeichnet, daß die schwach saure Pufferlösung einer Phosphorsalzlösung mit einem pH-Wert von 6,8 ist.

4. Verfahren gemäß Anspruch 2 oder 3, dadurch gekennzeichnet, daß die durch Auflösung der Schweinshautgelatine in der besagten Pufferlösung erzielte Lösung 6 Gewichts-% der besagten Gelätine enthält.

5. Verfahren gemäß eines der Ansprüche 2 bis 4, dadurch gekennzeichnet, daß man nach der Auflösung der Schweinshautgelatine die erhaltene Lösung, nach einer kurzen Inkubation bei einer Temperatur zwischen 25 und 50°C, auf einem geeigneten Träger verstreicht, von dem man sie nach Trocknung entfernt.

6. Verfahren gemäß Anspruch 5, dadurch gekennzeichnet, daß die besagte Inkubation während einer Stunde bei 37°C durchgeführt wird.

7. Verfahren gemäß Anspruch 5, dadurch gekennzeichnet, daß der geeignete Träger durch einen Polycarbonatfilm gebildet wird.

8. Verfahren gemäß irgendeines der Ansprüche 2 bis 7, dadurch gekennzeichnet, daß die Vernetzung der besagten homogenen Membrane durch eine 0,5-volumen-prozentige Glutaraldehyd-Lösung erfolgt.

9. Verfahren gemäß Anspruch 8, dadurch gekennzeichnet, daß der pH-Wert der besagten Glutaraldehyd-Lösung der gleiche ist wie der der besagten Pufferlösung.

10. Verfahen gemäß irgendeines der Ansprüche 2 bis 9, dadurch gekennzeichnet, daß die Sättigung der besagten vernetzten Membrane durch die Luciferase in Anwesenheit von Dithiothreit erfolgt.

**Claims**

1. A carrier membrane of luciferase especially for the dosaging of the acide adenosine triphosphate, characterised by the fact that the said membrane comprises gelatine of pigskin.

2. A method for the manufacture of a membrane according to Claim 1, characterised in that gelatine of pigskin is dissolved in a weakly acid buffer in order to obtain a homogeneous membrane, that the reticulation of the said homogeneous membrane is then carried out by means of a solution of glutaraldehyde at a weakly acid pH, and the membrane which is obtained is saturated with a quantity of luciferase.

3. A method according to Claim 1, characterised in that the weakly acid buffer is a solution of sodium phosphate, the pH of which is 6.8.

4. A method according to Claim 2 or 3, characterised in that the solution obtained by dissolving gelatine of pigskin into the said buffer comprises 6% by weight of the said gelatine.

5. A method according to one of Claims 2 to 4, characterised in that after the dissolving of the gelatine of pigskin, the solution which is obtained is spread out, after a brief incubation at a temperature of between 25 and 50°C, on a suitable support from which it will be removed after drying.

6. A method according to Claim 5, characterised in that the said incubation is carried out for one hour at 37°C.

7. A method according to Claim 5, characterised in that the suitable support is constituted by a film of polycarbonate.

8. A method according to any one of Claims 2 to 7, characterised in that the reticulation of the said homogeneous membrane is carried out with a solution having 0.5% by volume of glutaraldehyde.

9. A method according to Claim 8, characterised in that the pH of the said solution of glutaraldehyde is the same as that of the said buffer.

10. A method according to any one of Claims 2 to 9, characterised in that the saturation by luciferase of the said reticulated membrane is carried out in the presence of dithiothreitol.